**Europäisches Patentamt**

**European Patent Office**

**Office européen des brevets**

(19)

(11) Veröffentlichungsnummer: **0 002 473**
**B1**

(12) **EUROPÄISCHE PATENTSCHRIFT**

(45) Veröffentlichungstag der Patentschrift: **03.02.82**

(21) Anmeldenummer: **78101530.0**

(22) Anmeldetag: **04.12.78**

(51) Int. Cl.³: **C 07 C 31/18,**
**C 07 C 29/14,**
**C 07 C 29/00, C 08 G 18/32**

(54) Verfahren zur Herstellung von niedermolekularen Polyhydroxylverbindungen und deren Verwendung zur Herstellung von Polyurethankunststoffen.

(30) Priorität: **16.12.77 DE 2756270**

(43) Veröffentlichungstag der Anmeldung:
**27.06.79 Patentblatt 79/13**

(45) Bekanntmachung des Hinweises auf die Patenterteilung:
**03.02.82 Patentblatt 82/5**

(84) Benannte Vertragsstaaten:
**BE CH DE FR GB IT SE**

(56) Entgegenhaltungen:
**BE - A - 434 216**
**DE - C - 830 951**
**FR - A 1 234 335**
**FR - A - 1 335 323**
**US - A - 2 759 024**

(73) Patentinhaber: **BAYER AG**
**Zentralbereich Patente, Marken und Lizenzen**
**D-5090 Leverkusen 1, Bayerwerk (DE)**

(72) Erfinder: **Möhring, Edgar, Dr.**
**Hufer Weg 49a**
**D-5060 Bergisch-Gladbach 2 (DE)**
Erfinder: **Wagner, Kuno, Dr.**
**Am Kiesberg 8**
**D-5090 Leverkusen 1 (DE)**
Erfinder: **Müller, Hanns Peter, Dr.**
**Berta-von-Suttner-Strasse 40**
**D-5090 Leverkusen 1 (DE)**

Courier Press, Leamington Spa, England.

## Verfahren zur Herstellung von niedermolekularen Polyhydroxylverbindungen und deren Verwendung zur Herstellung von Polyurethankunstoffen

Die vorliegende Erfindung betrifft ein verbessertes Verfahren zur Herstellung von niedermolekularen Polyalkoholen durch katalytische Hydrierung eines Gemisches verschiedener niddermolekularer Hydroxyaldehyde, Hydroxyketone und gegebenenfalls mehrwertiger Alkohole, wie es bei der Selbstkondensation von Formaldehyd entsteht (im folgenden wird ein derartiges Gemisch als "Formose" bezeichnet). Die Erfindung betrifft auch die Verwendung dieser Polyalkohole für die Herstellung vor Polyurethankunststoffen.

Seit den Arbeiten von Butlerow und Loew (Ann. *120,* 295 (1861) und J. prakt. Chem. *33,* 321 (1886)) im vorigen Jahrhundert ist es bakannt, daß sich bei der Selbstkondensation des Formaldehydhydrats (Formosesynthese) unter dem Einfluß basischer Verbindungen, wie z.B. Calcium- oder Bleihydroxid, Hydroxyaldehyde und Hydroxyketone bilden. In der Folge wurde die Formosesynthese immer wieder bearbeitet. Beispielsweise seien in diesem Zusammenhang Pfeil, Chem. Berichte *84,* 229 (1951), Pfeil und Schroth, Chemische Berichte *85,* 303 (1952), R. D. Partridge und A. H. Weiss. Carbohydrate Research *24,* 29—44 (1972), die Formosen aus Glycerinaldehyd bzw. Dioxyaceton nach Emil Fischer, die deutschen Patentschriften 822,385, 830, 951 und 884,791, die US-Patentschrift 2 121 981, 2 224 910, 2 269 935 und 2 272 378 sowie die britische Patentschrift 513 708 genannt. Diese bekannten Verfahren des Standes der Technik sind mit gewissen Nachteilen behaftet (schlechte Raum/Zeit-Ausbeuten, gefärbte Nebenprodukte); in jüngster Zeit wurden jedoch von der Anmelderin neue Verfahren entwickelt, nach denen sich in hoher Ausbeute mit gängigen Katalysatoren praktisch farblose und von storenden Nebenprodukten freie Formosen herstellen lassen.

Eines dieser neuen Verfahren besteht darin, daß man die Kondensation des Formaldehydhydrats in Gegenwart von löslichen oder unlöslichen Blei(II)salzen, bzw. an hochmolekulare Träger gebundenen Blei(II)ionen als Katalysator und eines Gemisches aus Hydroxyaldehyden und Hydroxyketonen als Co-Katalysator ablaufen läßt, wie es bei der Kondensation von Formaldehydhydrat entsteht und welches durch folgende Molverhältnisse charakterisiert ist:

| | |
|---|---|
| Verbindungen mit 3 C-Atomen/Verbindungen mit 4 C-Atomen: | 0,5 bis 2,0 |
| Verbindungen mit 4 C-Atomen/Verbindungen mit 5 C-Atomen: | 0,2 bis 2,0 |
| Verbindungen mit 5 C-Atomen/Verbindungen mit 6 C-Atomen: | 0,5 bis 5,0 |

wobei der Anteil der Kimponenten mit 3 bis 6 C-Atomen mindestens 75 Gew.-%, vorzugsweise mehr als 85 Gew.-%, bezogen auf gesamten Co-Katalysator, beträgt.

Die Reaktionstemperatur liegt dabei im allgemeinen zwischen 70 und 110°C, bevorzugt zwischen 80 und 100°C, und der pH-Wert der Reaktionslösung wird durch kontrollierte Zugabe einer anorganischen oder organischen Base bis zu einem Umsatz von 10—60%, vorzugsweise 30—50%, auf einen Wert von 6,0—8,0, bevorzugt 6,5—7,0 und anschließend auf einen Wert von 4,0—6,0, bevorzugt 5,0—6,0 eingestellt. Überraschenderweise läßt sich die Produktverteilung der entsprechender Polyol-, Hydroxyaldehyd- und Hydroxyketon- gemische durch diese spezielle pH-Führung und durch anschließende Kühlung bei verschieden hohen Restformaldehydgehalten (0 bis 10 Gew.-%, vorzugsweise 0,5 bis 6 Gew.-%), in reproduzierbarer Weise variieren.

Nachdem man die Selbstkondensation des Formaldehydhydrates durch Kühlen und/oder durch Desaktivierung des bleihaltigen Katalysators mittels Säuren unterbrochen hat, wird der Katalysator und gegebenenfalls auch das in den Produkten enthaltende Wasser entfernt. Hinsichtlich näherer Einzelheiten sei auf DT—A 2 639 084 unde DT—A 2 732 077 verwiesen.

Eine weitere Möglichkeit, in hoher Raum-Zeit-Ausbeute hochkonzentrierte farblose Formosen herzustellen, besteht gemäß DT—A 2 714 084 darin, wäßrige Formalinlösungen und/oder Paraformaldehyd-Dispersionen in Gegenwart eines löslichen oder unlöslichen Metallkatalysators und eines durch teilweise Oxidation eines zwei- oder mehrwertigen, mindestens zwei benachbarte Hydroxylgruppen aufweisenden Alkohols mit einem Molekulargewicht zwischen 62 und 242 bzw. eines Gemisches derartiger Alkohole dargestellten Co-Katalysators zu kondensieren, wobei man den pH-Wert der Reaktionslösung durch gesteuerte Zufuhr einer Base bis zu einem Umsatz vor 5 bis 40% zwischen 6,0 und 9,0 hält und anschließend bis zum Abbruch der Kondensationsreaktion auf 4,5 bis 8,0 einstellt, so daß er nun um 1,0 bis 2,0 Einheiten tiefer liegt als in der ersten Reaktionsphase, dann die Reaktion bei einem Restgehalt von 0—10 Gew.-% Formaldehyd durch Desaktivierung des Katalysators unterbricht und den Katalysator entfernt.

Qualitativ hochwertige Formosen können auch durch Kondensation von Formaldehyd in Gegenwart eines Metallkatalysators und mehr als 10 Gew.-%, bezogen auf Formaldehyd, eines oder mehrerer zwei- oder mehrwertiger niedermolekularer Alkohcle und/oder höhermolekularer Polyhydroxylverbindungen hergestellt werden (siehe DT—A 2 714 104).

Besonders wirtschaftlich ist es, gemäß einem weiteren noch unveröffentlichten Verfahren der Anmelderin, Formose direkt, d.h. ohne den Umweg über wäßrige Formalinlösungen oder Paraformaldehyd, aus Formaldehyd enthaltenden Synthesegasen herzustellen.

# 0 002 473

Man leitet zu diesem Zweck die Synthesegase, wie sie bei der großtechnischen Herstellung von Formaldehyd anfallen, bei Temperaturen zwischen 10 und 150°C kontinuierlich oder diskontinuierlich in eine Absorptionsflüssigkeit, welche aus Wasser, ein- oder mehrwertigen niedermolekularen Alkoholen und/oder höhermolekularen Polyhydroxylverbindungen und/oder zur Endiolbildung befähigten Verbindungen als Co-Katalysator und/oder löslichen oder unlöslichen Metallverbindungen, welche gegebenenfalls an hochmolekulare Träger gebunden sind, als Katalysator besteht und einen pH-Wert von 3 bis 10 aufweist, kondensiert den Formaldehyd direkt in situ in der Absorptionsflüssigkeit (gegebenenfalls auch in einem nachgeschalteten Reaktionsrohr bzw. einer nachgeschalteten Rührkesselkaskade), bricht die Selbstkondensation des Formaldehyds bei einem Restformaldehydgehalt in Reaktionsgemisch von 0 bis 10 Gew.-% durch Kühlen und/oder durch Desaktivierung des Katalysators mittels Säuren ab und entfernt schließlich den Katalysator.

Gemische aus Hydroxyaldehyden, Hydroxyketonen und gegebenenfalls Polyalkoholen, wie sie nach den vorstehend beschriebenen Verfahren oder nach Verfahren des Standes der Technik erhalten werden, müssen für eine Reihe von Verwendungszwecken durch Reduktion der Carbonylgruppen in Gemische von Polyalkoholen umgewandelt werden (derartige, durch Reduktion von Formosen erhaltene Polyolgemische werden im folgenden "Formite" genannt). So gelingt z.B. die Reduktion von Formose direkt aus wäßriger Lösung schon bei Raumtemperatur mit Natriumborhydrid (vgl. R. D. Partridge, A. H. Weiss und D. Todd, Carbohydrate Research *24* (1972), 42); sie kann aber z.B. auch auf elektrochemischem Weg erfolgen.

Verfahren zur katalytischen Hydrierung von Zuckern, wie auch von Formose sind in großer Zahl bekannt. Dabei wird je nach Verfahrensweise mit sehr unterschiedlichen Mengen und Typen von Katalysatoren gearbeitet. So beschreiben L. Orthner und E. Gerisch (Biochem. Zeitung *259*, 30 (1933)) ein Verfahren zur katalytischen Hydrierung von Formose, bei dem in 7—8 stündiger Reaktion bei 130°C unter 120 bar Wasserstoffdruck eine 4 %ige wäßrige Formoselösung mit 170 Gew.-%, bezogen auf Formose, an Raney-Nickel hydriert wird. Ein solches Verfahren ist natürlich in jeder Hinsicht wirtschaftlich unbefriedigend. Aus der US-Patentschrift 2 269 935 ist ein Verfahren bekannt geworden, nach dem eine ca. 40 Gew.-% Formose enthaltende Lösung im sauren pH-Bereich mit 20 Gew.-% Nickelkatalysator bei 600 bis 620 bar Wasserstoffdruck und 120°C hydriert wird. Nachteilig an dieser Verfahrensvariante ist nicht nur der hohe Betriebsdruck, sondern auch der einzuhaltende niedrige pH-Wert, der zu durch Ni-Ionen grün gefärbten Produkten führt.

Aus der US-Patentschrift 2 224 910 ist ein Verfahren zur Hydrierung von Formose bekannt, bei dem eine 40 %ige Formoselösung mit 30 Gew.-% Raney-Nickel, bezogen auf Formose, bei 140 bis 210 bar $H_2$-Druck und pH 7 innerhalb von 4 Stunden hydriert wird. Auch dieses Verfahren befriedigt aufgrund des hohen Katalysatoraufwandes und der langen Reaktionszeit nicht.

Weitere Hydrierverfahren sind in den deutschen Patentschriften 705 274, 725 842, 830 951, 888 096 und 1 004 157 sowie in den US-Patentschriften 2 271 083, 2 272 378, 2 276 192, 2 760 983 und 2 775 621 beschrieben. Alle diese Verfahren weisen jedoch einen oder mehrere der folgenden Nachteile auf: großer apparativer Aufwand und schwierige Handhabung wegen hoher Wasserstoffdrucke; hoher Katalysatoraufwand, bezogen auf das hydrierte Produkt (10—200 Gew.-%); gefärbte Produkte durch lange Hydrierzeiten (1—10 Stunden).

Gemeinsam ist allen bisher bekannten Verfahren die Verwendung von Metall- oder gegebenenfalls Edelmetallkatalysatoren. Insbesondere wird Raney-Nickel eingesetzt, welches jedoch erst im alkalischen Bereich seine volle Aktivität entfaltet. So beschreibt die BE—A 434 216 ein Verfahren zur katalytischen Hydrierung von Substanzen, die durch Formaldehyd-Kondensation erhalten werden, in Gegenwart von restlichem Formaldehyd bei einem pH-Wert von 6,5 bis 10, erhöhtem Wasserstoffdruck und einer von 20—30°C auf 130—150°C ansteigenden Temperatur, wobei ein Katalysatoraufwand von mehr als 10 Gew.-%, bezogen auf die eingesetzten Hydroxyaldehyde und Hydroxyketone, erforderlich ist (Beispiel 1 der BE—A).

Da im alkalischen Milieu die Formose zur Karamelisierung neigt und stark verfärbte Produkte entstehen, wird nach einigen Verfahren des Standes der Technik im allgemeinen nicht im alkalischen, sondern im schwach sauren bzw. neutralen pH-Bereich gearbeitet. So arbeitet beispielsweise das in der FR—A 1 335 323 beschriebene Verfahren zur Herstellung von mehrwertigen Polyolen durch katalytische Hydrierung von Hydroxyaldehyden bei einem pH-Wert von weniger als 6,5, am besten zwischen 3 und 4, wobei eine Aldehydkonzentration von weniger als 0,05 Äquivalenten je Kilogramm Reaktionsgemisch aufrechterhalten wird.

Aufgabe der Erfindung war es daher, ein Verfahren zur schnellen Hydrierung von Formose mit geringem technischen Aufwand und sehr geringem Katalysatoraufwand bereitzustellen.

Überraschenderweise wurde nun gefunden, daß man- im Gegensatz zur in der Literatur bisher vertretenen Ansicht - Formose (gegebenenfalls in Gemisch mit anderen natürlichen oder synthetischen Zuckern) im alkalischen Milieu in schneller Reaktion, mit niedrigem Katalysatoraufwand, bei Wasserstoffdrücken zwischen 50 und 300 bar und Temperaturen zwischen 80 bis 220°C zu farblosen Polyolgemischen hydrieren kann. Dies ist neu und im Hinblick auf die oben erwähnten Karamelisierungsreaktionen der Formose, die bei erhöhter Temperatur im alkalischen Milieu besonders schnell ablaufen, völlig unerwartet. Vielmehr wären dunkle bis schwarze Produkte zu erwarten gewesen.

Es ist auch als überraschend anzusehen, daß bei den nach der erfindungsgemäßen Verfahren-

# 0 002 473

sweise erhaltenen Polyalkoholgemischen der Anteil an niedermolekularen $C_2$-, $C_3$- und $C_4$- Alkoholen wesentlich größer ist als bei nach üblichen Verfahren erhaltenen Formiten. Dies ist für eine Reihe von Anwendungsgebieten von besonderem Vorteil.

Gegenstand der vorliegenden Erfindung ist somit ein Verfahren zur Herstellung von niedermolekularen, mehrwertigen Alkoholen durch Reduktion von Formose bei einer Temperatur von 80 bis 220°C und unter einem Wasserstoffdruck von 50 bis 300 bar in Gegenwart eines Metallkatalysators bei einem pH-Wert von 7,5 bis 12,5, vorzugsweise von 8,5 bis 11,5, welches dadurch gekennzeichnet ist, daß man

(1) eine mindestens 20 %ige, bevorzugt mehr als 35 %ige, besonders bevorzugt mehr als 45 %ige Lösung von Formose chargenweise in einen—auf einer Temperatur von 80 bis 220°C, vorzugsweise 100 bis 200°C, besonders bevorzugt 140 bis 190°C, gehaltenen—Reaktor einbringt, so daß der Gehalt an reduzierbaren Gruppen (bestimmt als Carbonylgruppen) im Produktgemisch innerhalb des Reaktors eine West von 2 Gew.%, vorzugsweise 1 Gew.-%, besonders bevorzugt 0,5 Gew.-%, nicht überschrietet und daß jede Charge die 3- bis 30-fache Menge des in Reaktor vorhandenen Katalysators beträgt,

(2) der Katalysator stationär im Reaktor verbleibt und seine Menge $10^{-4}$ bis $5.10^{-2}$ Gewichtsteile pro 1 Gewichtsteil der Gesamtmenge sämtlicher Chargen an zu reduzierenden Ausgangsprodukten beträgt und

(3) das Reaktionsprodukt chargenweise aus dem Reaktor abzieht, nachdem der Gehalt an reduzierbaren Gruppen (bestimmt als Carbonylgruppen) unter 0,15 Gew.-%, vorzugsweise 0,05 Gew.-%, gesunken ist.

Das erfindungsgemäße Verfahren wird vorteilhaft in folgender Weise ausgeführt:

In einem Druckreaktor wird die Menge an Katalysator (bevorzugt Raney-Nickel) in Wasser vorgelegt, die zur Hydrierung des gesamten Ansatzes nötig ist. Dann wird der Reaktor mit Wasserstoffgas bis zum Betriebsdruck von 50 bis 300 bar gefüllt und anschließend auf die Hydriertemperatur von 80 bis 220°C aufgeheizt. Dann wird die drei- bis dreißigfache Menge (bevorzugt fünf- bis zwanzigfache) Menge Formoselösung bezogen auf Katalysator, langsam (d.h. etwa 1/6 des Reaktorvolumens in 3 Minuten bis 2 Stunden, vorzugsweise in 5 bis 30 Minuten) zugepumpt. Man läßt danach die halbe bis die vierfache Zeit, die zum Zupumpen benötigt wurde, nachhydrieren. Anschließend drückt man die Menge Reaktionsgemisch, die der zugepumpten Menge Formoselösung entspricht, über eine Stahlmantelfritte ab, wobei der Katalysator im Reaktor verbleibt. Anschließend wird eine neue Charge in den Reaktor gepumpt und mit dieser ebenso verfahren wie mit der ersten Charge. Alle weiteren Chargen werden in gleicher Weise behandelt.

Diese erfindungsgemäße chargenweise Pumphydrierung erbringt extrem hole Katalysatorstandzeiten und somit—bezogen auf die Gesamtmenge an zu reduzierender bzw. an reduzierter Formoseeinen sehr kleinen Katalysatoraufwand.

Es sei in diesem Zusammenhang noch einmal darauf hingewiessen, daß die erfindungsgemäß möglichen hohen Katalysatorstandzeiten völlig überraschend sind, da eigentlich aufgrund der im alkalischen Milieu an der Formose zu erwartenden Karamelisierungs-reaktionen damit zu rechnen gewesen wäre, daß die entstehenden Produkte den Katalysator desaktivieren würden.

Das erfindungsgemäße Verfahren zur Herstellung von hydrierten Zuckern oder hydrierter Formose ("Formit") bietet gegenüber dem Stand der Technik folgende wesentliche Vorteile:

1. Das erfindungsgemäße Verfahren ist äußerst wirtschaftlich; verglichen mit allen bisher bekannten Verfahren ist nur ein äußerst geringer Katalysatoraufwand nötig; die Hydriergeschwindigkeit ist sehr hoch, so werden beispielsweise bei hoher Temperatur (150—190°C) pro hydrierschritt nur 10 bis 15 Minuten benötigt.

2. Das erfindungsgemäße Verfahren läßt sich ohne großen technischen Aufwand durchführen, da wegen der relative niedrigen Drücke keine Spezialapparaturen verwendet und Sicherungsmaßnahmen getroffen werden müssen.

3. Das erfindungsgemäße Verfahren liefert hellfarbige bis farblose Produkte, die für die verschiedenartigsten Anwendungszwecke ohne weitere Reinigungsschritte eingesetzt werden können.

4. Ein überraschender und besonderer Vorzug des Verfahrens ist die weitgehende Spaltung der Ausgangssubstanzen in niedermolekulare $C_2$- bis $C_5$-Alkohole, wodurch die Viskosität der Polyhydroxylverbindungen gesenkt, ihre Verarbeitbarkeit verbessert und gleichzeitig die Verträglichkeit gegenüber anderen Substanzen erhöht wird, speziell mit den bei der Herstellung von Kunststoffen nach dem Polyisocyanat-Polyadditionsverfahren verwendeten Ausgangskomponenten (insbesondere höhermolekulare Polyhydroxyverbindungen und Treibmittel).

5. Weiterhin ist es besonders vorteilhaft, daß gewünschtenfalls weiter Verbindungen wie z.B. Alkanale, Alkohole (z.B. $C_1$- bis $C_{23}$-, insbesondere $C_1$- bis $C_8$-Alkohole, wobeil diese Alkohole ein- oder mehrwertig sein können), Ketone, Aldehyde oder höhermolekulare Polyole bei der Hydrierung zugegen sein können, da diese insbesondere die Verträglichkeit der Reaktionsprodukte gegenüber den beim Polyisocyanat-Polyadditionsverfahren verwendeten

4

Treibmitteln verbessern. Diese Aldehyde, ketone und Alkanale können in Mengen bis zu 50 Gew.-% (bezogen auf Gesamtmenge der zu reduzierenden Produkte) zugesetzt werden.

6. Weiterhin lassen sich nach dem erfindungsgemäßen Verfahren neben Formose andere natürliche und künstliche Zucker hydrieren.

Als Aldehyde bzw. Alkanale, die im erfindungsgemäßen Verfahren gegebenenfalls mitverwendet werden können kommen insbesondere Acetaldehyd, Propionaldehyd, Butyraldehyd, Isobutyraldehyd sowie deren Methylolderivate in Frage.

Als Ketone seien Aceton, Methyläthylketon, Diäthylketon, Cyclopentanon, Cyclohexanon, Mesityloxid, Isophoron, Acetophenon, Benzophenon sowie deren Methylolderivate genannt.

Als Lösungsmittel kommt im erfindungsgemäßen Verfahren in erster Linie Wasser in Frage; die Formose kann jedoch auch in beliebigen Mono- oder Polyalkoholen gelöst sein. Geeignete Alkohole sind z.B. Methanol, Äthanol, Propanol, Butanol, Isopropanol, Isobutanol, Cyclopentanol, Cyclohexanol, 2 - Äthoxyäthanol, 2 - Propoxyäthanol, 2 - isopropoxyäthanol, 2 - Butoxyäthanol, 2 - (2 - Methoxyäthoxy) - äthanol), 2 - (2 - Äthoxyäthoxy) - äthanol, 1,2 - Bis(2 - hydroxyäthoxy) - äthan, Äthylenglykol, Diäthylenglykol, Triäthylenglykol, Tetraäthylenglykol, 1,2 - Propandiol, Isopropylenglykol, 1,3 - Propandiol, 1,2 - Butandiol, 1,3 - Butandiol, 2 - Methoxy - 1 - butanol, 2,3 - Butandiol, 1,5 - Pentadiol, 2,2 - Dimethyl - 1,3 - propandiol, 1,6 - Hexandiol, 2,5 - Hexandiol, 2 - Methyl - 2,4 - pentandiol, 3 - Methyl - 1,5 - pentandiol, 3 - Methyl - 2,4 - pentandiol, 2,3 - Dimethyl - 2,3 - butandiol, 2 - Methyl - 2 - propyl - 1,3 - propandiol, 2,2 - Diäthyl - 1,3 - propandiol, 2 - Äthyl - 1,3 - hexandiol, 2,5 - Dimethyl - 2,5 - hexandiol, 2,2,4 - Trimethyl - 1,3 - pentandiol, 1,3 - Diäthoxy - 2 - propanol, 2 - Hydroxymethyl 2 - methyl - 1,3 - propandiol, 1,2,6 - Hexantriol, 2 - Äthyl - 2 - hydroxymethyl - 1,3 - propandiol, 2,2 - Bis - hydroxymethyl - 1,3 - propandiol Erythrit, Chinit, Mannit, Sorbit und Methylglykosid, sowie Athoxylierungs- und Propoxylierungsprodukte dieser Alkohole mit einem Molekulargewicht bis ca. 400 und selbstverständlich auch Gemische dieser Alkohole. Besonders bevorzugt sind Äthylenglykol, Glycerin und 1,4-Butandiol.

Erfindungsgemäß können bei der Formosehydrierung—gegebenenfalls im Gemisch mit den obengenannten Alkoholten—jedoch auch Polyhydroxylverbindungen mit einem Molekulargewicht von 400 bis 10 000, vorzugsweise 500 bis 6000, mitverwendet werden, welche zweckmäßigerweise ebenfalls bei Raumtemperatur flüssig bzw. in der Formoselösung löslich sind, z.B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind.

Das erfindungsgemäße Hydrierverfahren ist auf beliebige Formosen anwendbar, wie sie bei den Verfahren des eingangs zitierten Standes der Technik erhalten werden. Die Formose kann jedoch auch im Gemisch mit bis zu 80 Gew.-% (bezogen auf Gesamtmenge an zu hydrierenden Verbindungen) an anderen künstlichen oder auch natürlichen Zuckern (wie z.B. Glucose, Maltose, Fructose, Saccharose, Lactose etc.) eingesetzt werden. Es ist dabei von Vorteil, daß Formose ein ausgezeichnetes Lösungsmittel bzw. einen Löslichkeitsvermittler für derartige Zucker darstellt.

Als erfindungsgemäß mitverwendbare künstliche Invertzucker kommen Hydrolysate von beliebigen Di- und/oder Polysacchariden in Frage, z.B. von Rohrzucker, Mischungen von Rohrzucker und Invertzuckern, Hydrolysate von Trehalose, Maltose oder Isomaltose, Hydrolysate von Mais- und Kartoffelstärke und von Pektinstoffen (Amylose und Amylopektine), Cellobiose und Lactose (Milchzucker), Hydrolysate von Galaktose, Glukosemischungen, Raffinose-Hydrolysate, Cellulose-Hydrolysate, Hydrolysate von Dextrinen, gegebenenfalls in Mischung mit nichthydrolysierten Dextrinen, Hydrolysate der Schardinger-Dextrine (cyclische Dextrine), Hydrolysate des Glykogens, Hydrolysate der Glukose-6-Phosphorsäure, Hydrolysate von Glukose-1-Phosphat (Cori-Ester), Fruktose-6-Phosphat, abgebaute Pektinstoffe (Polygalakturonsäuren), abgebaute Glukosamine, Hydrolysate von Melasserückständen etc.

Der pH-Wert der zu hydrierenden Lösung kann sowohl mit anorganischen als auch mit organischen Basen eingestellt werden. Bevorzugt sind Natriumhydroxid, Kaliumhydroxid, Calciumhydroxid, Magnesiumhydroxid, Bariumhydroxid, Aluminiumoxidhydrat, Triäthylamin, N-Methylmorpholin und N-Methylpiperidin.

Besonders bevorzugt ist jewails die bei der Formosesynthese verwendete Base, die vor der Hydrierung durch Behandeln der Formoselösung mit in der OH-Form vorliegendem Ionenaustauscherharz in die freie OH-Form übergeführt wird, wodurch sich die benötigte Alkalität der Formoselösung von selbst einstellt.

Als Hydrier-Katalysatoren kommen für das erfindungsgemäße Verfahren insbesondere Metalle mit den Ordnungszahlen 23 bis 29 (in elementarer und/oder oxidischer Form) in Frage. Geeignete Katalysatoren sind beispielsweise solche auf Basis von Nickel oder Kobalt, wobei als Träger für den Katalysator sowohl anorganische Materialien wie Kieselgur, Kieselsäuren, Aluminiumoxide, Alkali- und Erdalkalisilikate, Aluminiumsilikate, Montmorillonit, Zeolithe, Spinelle, Dolomit, Kaolin, Magnesiumsilikate, Zirkonoxid, Eisenoxid, Zinkoxid, Calciumcarbonat, Siliziumcarbid, Aluminiumphosphat, Borphosphat, Asbest oder Aktiv-Kohle als auch organische Materialien, z.B. natürlich vorkommende oder synthetische Verbindungen mit hohem Molgewicht wie Seide, Polyamide, Polystyrole, Zellstoff oder Polyurethane verwendbar sind; das Trägermaterial kann dabei z.B. in Form

5

von Kugeln, Strängen, Fäden, Zylindern, Polygonen oder in Pulverform vorliegen. Bevorzugt sind Raney-Typ-Katalysatoren, wie Raney-Nickel, W-1-, W-5-, W-6- und W-7- Raney-Nickel (siehe H. Adkins, J. Am. Chem. Soc. *69*, 3039 (1974)), Raney-Kobalt-Katalysatoren, Raney-Kupfer, Raney-Nickel-Eisen, Raney-Kobalt-Nickel und Raney-Kobalt-Eisen. In Betracht kommen auch durch Reduktion von Nickel- oder Kobaltsalzen hergestellte Metallkatalysatoren, wie Urushibara-Nickel oder mit Metallalkylverbindungen, Alkalihydriden, Hydrazin, Boranaten oder Borwasserstoff reduzierte Nickel- oder Kobaltsalze durch Reduktion der Metalloxide oder Metalloxidgemische hergestellte Katalysatoren, aber auch die Metalloxide oder -oxidgemische selbst.

Die erfindungsgemäß bevorzugten Katalysatoren auf Basis der Metalle mit den Ordnungszahlen 23—29 können als Beschleuniger eines oder mehrere der folgenden Elemente in Mengen bis zu 10 Gew.-% enthalten:

Li, Na, Ca, Ba, K, Ag, Be, La, Ce, V, Nb, Ta, Mo, W und bis zu 1 Gew.-% der Elemente Ru, Rh, Pd, Au, Ir, Pt.

Besonders geeignete Katalysatoren sind Raney-Nickel mit 90 Gew.-% Ni und <1 Gew.-% Fe, Ca und Na, Raney-Nickel-Eisen mit 5 bis 30 Gew.-% Fe und <1 Gew.-% Ca und Na und Raney-Kobalt-Eisen mit 10—30 Gew.-% Fe.

Bevorzugter Verwendungszweck der erfindungsgemäß hergestellten Gemische von mehrwertigen, niedermolekularen Alkoholen ist ihr Einsatz als Polyolkomponente im Polyisocyanat-Polyadditionsverfahren.

Gegenstand der vorliegenden Erfindung ist somit auch die Verwendung der niedermolekularen mehrwertigen Alkohole zur Herstellung von gegebenenfalls zellförmigen Polyurethankunststoffen durch Umsetzung von

(a) Polyisocyanaten mit

b) mindestens zwei aktive Wasserstoffatome aufweisenden Verbindungen mit einem Molekulargewicht zwischen 32 und 400, gegebenenfalls

c) mindestens zwei aktive Wasserstoffatome aufweisenden Verbindungen mit einem Molekulargewicht zwischen 400 und 10 000 sowie gegebenenfalls.

d) Treibmitteln, Katalysatoren und weiteren an sich bekannten Zusatzstoffen,

welche dadurch gekennzeichnet ist, daß als Komponente b), gegebenenfalls nur anteilsweise, erfindungsgemäß hergestellte Gemische niedermolekularer, mehrwertiger Alkohole eingesetzt werden.

Als erfindungsgemäß einzusetzende Ausgangskomponenten kommen aliphatische, cycloaliphatische, araliphatische, aromatische und heterocyclische Polyisocyanate in Betracht, wie sie z. B. von W. Siefken in Justus Liebigs Annalen der Chemie, 562, Seiten 75 bis 136, beschrieben werden, beispielsweise Äthylen-diisocyanat, 1,4-Tetramethylendiisocyanat, 1,6-Hexamethylendiisocyanat, 1,12-Dodecandiisocyanat, Cyclobutan - 1,3 - diisocyanat, Cyclohexan - 1,3- und -1,4 - diisocyanat sowie beliebige Gemische dieser Isomeren, 1 - Isocyanato - 3,3,5 - trimethyl - 5 - isocyanatomethyl - cyclohexan (DAS 1 202 785, amerikanische Patentschrift 3 401 190), 2,4- und 2,6 - Hexahydrotoluylendiisocyanat sowie beliebige Gemische dieser Isomeren, Hexahydro - 1,3- und/oder 1,4 - phenylen - diisocyanat, Perhydro - 2,4'- und/oder -4,4' - diphenylmethan - diisocyanat, 1,3- und 1,4 - Phenylendiisocyanat, 2,4- und 2,6 - Toluylendiisocyanat sowie beliebige Gemische dieser Isomeren, Diphenylmethan - 2,4'- und/oder -4,4' - diisocyanat, Naphthylen - 1,5 - diisocyanat, Triphenylmethan - 4,4',4'' - triisocyanat. Polyphenyl - polymethylen - polyisocyanate, wie sie durch Anilin - Formaldehyd - Kondensation und anschließende Phosgenierung erhalten und z.B. in den britischen Patentschriften 874 430 und 848 671 beschrieben werden, m- und p-Isocyanatophenyl - sulfonyl - isocyanate gemäß der amerikanischen Patentschrift 3 454 606, perchlorierte Arylpolyisocyanate, wie sie z.B. in der deutschen Auslegeschrift 1 157 601 (amerikanische Patentschrift 3 277 138) beschrieben werden, Carbodiimidgruppen aufweisende Polyisocyanate, wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der Veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 002 789, 1 222 067 und 1 027 394 sowie in den deutschen Offenlegungsschriften 1 929 034 und 2 004 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der deutschen Patentschrift 1 101 394 (amerikanische Patentschriften 3 124 605 und 3 201 272) sowie in der britischen Patentschrift 889 050 beschrieben werden, durch Telomerisationsreaktionen hergestellte Polyisocyanate, wie sie z.B. in der amerikanischen patentschrift 3 654 106 beschrieben werden, Estergruppen aufweisende Polyisocyanate, wie sie zum Beispiel in den britischen patentschriften 965 474 und 1 072 956, in der amerikanischen Patentschrift 3 567 763 und in der deutschen Patentschrift 1 231 688 genannt werden, Umsetzungsprodukte der obengenannten Isocyanate mit

**0 002 473**

Acetalen gemäß der deutschen Patentschrift 1 072 385 und polymer Fettsäurereste enthaltende Polyisocyanate gemäß der amerikanischen Patentschrift 3 455 883.

Es ist auch möglich, die bei der technischen Isocyanatherstellung anfallenden, Isocyanatgruppen aufweisenden Destillationsrückstände, gegebenenfalls gelöst in einem oder mehreren der vorgenannten Polyisocyanate, einzusetzen. Ferner ist es möglich, beliebige Mischungen der vorgenannten Polyisocyanate zu verwenden.

Besonders bevorzugt werden in der Regal die technisch leicht zugänglichen Polyisocyanate, z.B. das 2,4- und 2,6 - Toluylendiisocyanat sowie beliebige Gemische dieser Isomeren ("TDI"), Polyphenyl - polymethylen - polyisocyanate, wie sie durch Anilin - Formaldehyd - Kondensation und anschließende Phosgenierung hergestellt werden ("rohes MDI") und Carbodiimidgruppen, Urethangruppen, Allophanatgruppen, Isocyanuratgruppen, Harstoffgruppen, oder Biuretgruppen aufweisenden Polyisocyanate ("modifizierte Polyisocyanate").

Erfindungsgemäß gegebenenfalls einzusetzende Ausgangskomponenten sind ferner Verbindungen mit mindestens zwei gegenüber Isocyanaten reaktionsfähigen Wasserstoffatomen von einem Molekulargewicht in der Regel von 400—10 000. Hierunter versteht man neben Aminogruppen, Thiolgruppen oder Carboxylgruppen aufweisenden Verbindungen vorzugsweise Polyhydroxylverbindungen, insbesondere zwei bis acht Hydroxylgruppen aufweisende Verbindungen, speziell solche vom Molekulargewicht 800 bis 10 000, vorzugsweise 1000 bis 6000, z.B. mindestens zwei, in der Regel 2 bis 8, vorzugsweise aber 2 bis 4, Hydroxylgruppen aufweisende Polyester, Polyäther, Polythioäther, Polyacetale, Polycarbonate und Polyesteramide, wie sie für die Herstellung von homogenen und von zellförmigen Polyurethanen an sich bekannt sind.

Die in Frage kommenden Hydroxylgruppen aufweisenden Polyester sind z.B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z.B. durch Halogenatome, substituiert und/oder ungesättigt sein.

Als Beispiele hierfür seien genannt: Bernsteinsäure, Adipinsäure, Korksäure, Azelainsäure, Sebacinsäure, Phthalsäure, Isophthalsäure, Trimellitsäure, Phthalsäureanhydrid, Tetrahydrophthalsäureanhydrid, Hexahydrophthalsäureanhydrid, Tetrachlorphthalsäureanhydrid, Endomethylentetrahydrophthalsäureanhydrid, Glutarsäureanhydrid, Maleinsäure, Maleinsäureanhydrid, Fumarsäure, dimere und trimere Fettsäuren wie Ölsäure, gegebenenfalls in Mischung mit monomeren Fettsäuren, Terephthalsäuredimethylester und Terephthalsäure-bis-glykolester. Als mehrwertige Alkohole kommen z.B. Äthylenglykol, Propylenglykol-(1,2) und -(1,3), Butylenglykol-(1,4) und -(2,3), Hexandiol-(1,6), Octandiol-(1,8), Neopentylglykol, Cyclohexandimethanol(1,4 - Bis - hydroxymethylcyclohexan), 2 - Methyl - 1,3 - propandiol, Glycerin, Trimethylolpropan, Hexantriol - (1,2,6), Butantriol - (1,2,4), Trimethyloläthan, Pentaerythrit, Chinit, Mannit und Sorbit, Methylglykosid, ferner Diäthylenglykol, Triäthylenglykol, Tetraäthylenglykol, Polyäthylenglykole, Dipropylenglykol, Polypropylenglykole, Dibutylenglykol und Polybutylenglykole in Frage. Die Polyester können anteilig endständige Carboxylgruppen aufweisen. Auch polyester aus Lactonen, z.B. ε-Caprolacton oder Hydroxycarbonsäuren, z.B. ω-Hydroxycapronsäure, sind einsetzbar.

Auch die erfindungsgemäß in Frage kommenden, mindestens zwei, in der Regel zwei bis acht, vorzugsweise zwei bis drei, Hydroxylgruppen aufweisenden Polyäther sind solche der an sich bekannten Art und werden z.B. durch Polymerisation von Epoxiden wie Äthylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole, Ammoniak oder Amine, z.B. Äthylenglykol, Propylenglykol-(1,3) oder -(1,2), Trimethylolpropan, 4,4' - Dihydroxy - diphenylpropan, Anilin, Äthanolamin oder Äthylendiamin hergestellt. Auch Sucrosepolyäther, wie sie z.B. in den deutschen Auslegeschriften 1 176 358 und 1 064 938 beschrieben werden, kommen erfindungsgemäß in Frage. Vielfach sind solche Polyäther bevorzugt, die überwiegend (bis zu 90 Gew.-%, bezogen auf alle vorhandenen OH-Gruppen im Polyäther) primäre OH-Gruppen aufweisen. Auch durch Vinylpolymerisate modifizierte Polyäther, wie sie z.B. durch Polymerisation von Styrol und Acrylnitril in Gegenwart von Polyäthern entstehen (amerikanische Patentschriften 3 383 351, 3 304 273, 3 523 093, 3 110 695, deutsche Patentschrift 1 152 536), sind geeignet, ebenso OH-Gruppen aufweisende Polybutadiene.

Unter den Polythioäthern seien insbesondere die Kondensationsprodukte von Thiodiglykol mit sich selbst und/oder mit anderen Glykolen, Dicarbonsäuren, Formaldehyd, Aminocarbonsäuren oder Aminoalkoholen angeführt. Je nach den Co-Komponenten handelt es sich bei den Produkten um Polythiomischäther, Polythioätherester oder Polythioätheresteramide.

Als Polyacetale kommen z.B. die aus Glykolen wie Diäthylenglykol, Triäthylenglykol, 4,4'-Dioxäthoxydiphenyldimethylmethan, Hexandiol und Formaldehyd herstellbaren Verbindungen in Frage.

Auch durch Polymerisation cyclischer Acetale lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Diäthylenglykol, Triäthylenglykol oder Tetraäthylenglykol mit Diarylcarbonaten, z.B. Diphenylcarbonat, oder Phosgen hergestellt werden können.

Zu den Polyesteramiden und Polyamiden zählen z.B. die aus mehrwertigen gesättigten und ungesättigten Carbonsäuren bzw. deren Anhydriden und mehrwertigen gesättigten und ungesättigten Aminoalkoholen, Diaminen, Polyaminen und ihren Mischungen gewonnenen, vorwiegend linearen Kondensate.

Auch bereits Urethan- oder Harnstoffgruppen enthaltende Polyhydroxylverbindungen sowie gegebenenfalls modifizierte natürliche Polyole, wie Rizinusöl, Kohlenhydrate oder Stärke, sind verwendbar. Auch Anlagerungsprodukte von Alkylenoxiden an Phenol-Formaldehyd-Harze oder auch an Harnstoff-Formaldehydharze sind erfindungsgemäß einsetzbar.

Vertreter dieser erfindungsgemäß ggf. zu verwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32—42 und Seiten 44—54 und Band II, 1964, Seiten 5—6 und 198—199, sowie im Kunststoff-Handbuch, Band VII, Vieweg-Höchtlen, Carl-Hanser-Verlag, München, 1966, z.B. auf den Seiten 45—71, beschrieben.

Selbstverständlich können Mischungen der obengenannten Verbindungen mit mindestens zwei gegunüber Isocyanaten reaktionsfähigen Wasserstoffatomen mit einem Molekulargewicht von 400—10 000, z.B. Mischungen von Polyäthern und Polyestern, eingesetzt werden.

Als erfindungsgemäß gegebenenfalls einzusetzende Ausgangskomponenten kommen auch Verbindungen mit mindestens zwei gegenüber Isocyanaten reaktionsfähigen Wasserstoffatomen von einem Molekulargewicht 32—400 in Frage. Auch in diesem Fall versteht man hierunter Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8 gegenüber Isocyanaten reaktionsfähige Wasserstoffatome auf, vorzugsweise 2 oder 3 reaktionsfähige Wasserstoffatome.

Als Beispiele für derartige Verbindungen seien genannt: Äthylenglykol, Propylenglykol(1,2) und-(1,3), Butylenglykol-(1,4) und -(2,3), Pentandiol-(1,5), Hexandiol-(1,6), Octandiol-(1,8), Neopentylglykol, 1,4 - Bishydroxymethyl - cyclohexan, 2 - Methyl - 1,3 - propandiol, Glyzerin, Trimethylolpropan, Hexantriol-(1,2,6), Trimethyloläthan, Pentaerythrit, Chinit, Mannit und Sorbit, Diäthylenglykol, Triäthylenglykol, Tetraäthylenglykol, Polyäthylenglykole mit einem Molekulargewicht bis 400, Dipropylenglykol, Polypropylenglykole mit einem Molekulargewicht bis 400, Dibutylenglykol, Polybutylenglykole mit einem Molekulargewicht bis 400, 4,4'-Dihydroxydiphenylpropan, Di - hydroxymethyl - hydrochinon, Äthanolamin, Diäthanolamin, Triäthanolamin, 3-Aminopropanol, Äthylendiamin, 1,3 - Diaminopropan, 1 - Mercapto - 3 - amino - propan, 4 - Hydroxy- oder -Amino - phthalsäure, Bernsteinsäure, Adipinsäure, Hydrazin, N,N' - Dimethylhydrazin, 4,4' - Diaminodiphenylmethan, Toluylendiamin, Methylenbischloranilin, Methylen - bis - anthranilsäureester Diaminobenzoesäureester und die isomeren Chlorphenylendiamine.

Auch in diesem Fall können Mischungen von verschiedenen Verbindungen mit mindestens zwei gegenüber Isocyanaten reaktionsfähigen Wasserstoffatomen mit einem Molekulargewicht von 32—400 verwendet werden.

Erfindungsgemäß können jedoch auch Polyhydroxylverbindungen eingesetzt werden, in welchen hochmolekulare Polyaddukte bzw. Polykondensate in feindisperser oder gelöster Form enthalten sind. Derartige modifizierte Polyhydroxylverbindungen werden erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) direkt in situ in den oben genannten, Hydroxylgruppen aufweisenden Verbindungen ablaufen läßt. Derartige Verfahren sind beispielsweise in den Deutschen Auslegeschriften 1 168 075 und 1 260 142, sowie den Deutschen Offenlegungsschriften 2 324 134, 2 423 984, 2 512 385, 2 513 815, 2 550 796, 2 550 797, 2 550 833 und 2 550 862 beschrieben. Es ist aber auch möglich, gemäß US-Patent 3 869 413 bzw. Deutscher Offenlegungsschrift 2 550 860 eine fertige wäßrige Polymerdispersion mit einer Polyhydroxylverbindung zu vermischen und anschließend aus dem Gemisch das Wasser zu entfernen.

Bei der Verwendung von modifizierten Polyhydroxylverbindungen der oben genannten Art als Ausgangskomponente in Polyisocyanat-Polyadditionsverfahren entstehen in vielen Fällen Polyurethankunststoffe mit wesentlich verbesserten mechanischen Eigenschaften.

Erfindungsgemäß können gegebenenfalls Wasser und/oder leicht flüchtige organische Substanzen als Treibmittel mitverwendet werden. Als organische Treibmittel kommen z.B. Aceton, Äthylacetat, halogensubstituierte Alkane wie Methylenchlorid, Chloroform, Äthyliden-chlorid, Vinylidenchlorid, Monofluortrichlormethan, Chlordifluormethan, Dichlordifluormethan, ferner Butan, Hexan, Heptan oder Diäthyläther infrage. Eine Treibwirkung kann auch durch Zusatz von bei Temperaturen über Raumtemperatur unter Abspaltung von Gasan, beispielsweise von Stickstoff, sich zersetzenden Verbindungen, z.B. Azoverbindungen wie Azoisobuttersäurenitril, erzielt werden. Weitere Beispiele für Treibmittel sowie Einzelheiten über die Verwendung von Treibmitteln sind im Kunststoff-Handbuch, Band VII, herausgegeben von Vieweg und Höchtlen, Carl-Hanser-Verlag, München 1966, z.B. auf den Seiten 108 und 109, 453 bis 455 und 507 bis 510 beschrieben.

Erfindungsgemäß werden ferner oft Katalysatoren mitverwendet. Als mitzuverwendende Katalysatoren kommen solche der an sich bekannten Art infrage, z.B. tertiäre Amine, wie Triäthylamin, Tributylamin, N - Methyl - morpholin, N - Äthyl - morpholin, N - Cocomorpholin, N,N,N',N' - Tetramethyl - äthylendiamin, 1,4 - Diaza - bicyclo - (2,2,2) - octan, N - Methyl - N' - diamethylaminoäthyl - piperazin, N,N - Dimethylbenzylamin, Bis - (N,N - diäthylaminoäthyl) - adipat, N,N - Diäthylbenzylamin, Pentamethyldiäthylentriamin, N,N - Dimethylcyclohexylamin, N,N,N',N' - Tetramethyl - 1,3 - butandiamin, N,N - Dimethyl - ß - phenyläthylamin, 1,2 - Dimethylimidazol, 2 - Methylimidazol. Als Katalysatoren kommen auch an sich bekannte Mannichbasen aus sekundären Aminen, wie Dimethylamin, und Aldehyden, vorzugsweise Formaldehyd, oder Ketonen wie Aceton, Methyläthylketon oder Cyclohexanon und Phenolen, wie Phenol, Nonylphenol oder Bisphenol in Frage.

Gegenüber Isocyanatgruppen aktive Wasserstoffatome aufweisende tertiäre Amine als Katalysatoren sind z.B. Triäthanolamin, Triisopropanolamin, N-Methyldiäthanolamin, N - Athyl - diäthanolamin, N,N - Dimethyl - äthanolamin, sowie deren Umsetzungsprodukte mit Alkylenoxiden, wie Propylenoxid und/oder Äthylenoxid.

Als Katalysatoren kommen ferner Silaamine mit Kohlenstoff - Silizium - Bindungen, wie sie z.B. in der deutschen Patentschrift 1 229 290 (entsprechend der amerikanischen Patentschrift 3 620 984) beschrieben sind, in Frage, z.B. 2,2,4 - Trimethyl - 2 - silamorpholin und 1,3 - Diäthylaminomethyl - tetramethyl - disiloxan.

Als Katalysatoren kommen auch stickstoffhaltige Basen wie Tetraalkylammoniumhydroxide, ferner Alkalihydroxide wie Natriumhydroxid, Alkaliphenolate wie Natriumphenolat oder Alkalialkoholate wie Natriummethylat in Betracht. Auch Hexahydrotriazine können als Katalysatoren eingesetzt werden.

Erfindungsgemäß können auch organische Metallverbindungen, insbesondere organische Zinnverbindungen, als Katalysatoren, verwendet werden.

Als organische Zinnverbindungen kommen vorzugsweise Zinn(II)-salze von Carbonsäuren wie Zinn(II)-acetat, Zinn(II)-octoat, Zinn(II)-äthylhexoat und Zinn(II)-laurat und die Zinn(IV)-Verbindungen, z.B. Dibutylzinnoxid, Dibutylzinndichlorid, Dibutylzinndiacetat, Dibutylzinndilaurat, Dibutylzinnmaleat oder Dioctylzinndiacetat in Betracht. Selbstverständlich können alle obengenannten Katalysatoren als Gemische eingesetzt werden.

Weitere Vertreter von erfindungsgemäß zu verwendenden (Katalysatoren sowie Einzelheiten über die Wirkungsweise der Katalysatoren sind im Kunststoff-Handbuch, Band VII, herausgegeben von Vieweg und Höchtlen, Carl-Hanser-Verlag, München 1966, z.B. auf den Seiten 96 bis 102 beschrieben.

Die Katalysatoren werden in der Regel in einer Menge zwischen etwa 0,001 und 10 Gew.-%, bezogen auf Formit, eingesetzt.

Erfindungsgemäß können auch oberflächenaktive Zusatzstoffe, wie Emulgatoren und Schaumstabilisatoren, mitverwendet werden. Als Emulgatoren kommen z.B. die Natriumsalze von Ricinusölsulfonaten oder Salze von Fettsäuren mit Aminen wie ölsaures Diäthylamin oder stearinsaures Diäthanolamin infrage. Auch Alkali-oder Ammoniumsalze von Sulfonsäuren wie etwa von Dodecylbenzolsulfonsäure oder Dinaphthylmethandisulfonsäure oder von Fettsäuren wie Ricinolsäure oder von polymeren Fettsäuren können als oberflächenaktive Zusatzstoffe mitverwendet werden.

Als Schaumstabilisatoren kommen vor allem Polyäthersiloxane, speziell wasserlösliche Vertreter, infrage. Diese Verbindungen sind im allgemeinen so aufgebaut, daß ein Copolymerisat aus Äthylenoxid und Propylenoxid mit einem Polydimethylsiloxanrest verbunden ist. Derartige Schaumstabilisatoren sind z.B. in den amerikanischen Patentschriften 2 834 748, 2 917 480 und 3 629 308 beschrieben.

Erfindungsgemäß können ferner auch Reaktionsverzögerer, z.B. sauerreagierende Stoffe wie Salzsäure oder organische Säurehalogenide, ferner Zellregler der an sich bekannten Art wie Paraffine oder Fettalkohole oder Dimethylpolysiloxane sowie Pigments oder Farbstoffe und Flammschutzmittel der an sich bekannten Art, z.B. Tris-chloräthylphosphat, Trikresylphosphat oder Ammoniumphosphat und -polyphosphat, ferner Stabilisatoren gegen Alterungs- und Witterungseinflüsse, Weichmacher und fungistatisch und bakteriotatisch wirkende Substanzen sowie Füllstoffe wie Bariumsulfat, Kieselgur, Ruß oder Schlämmkreide mitverwendet werden.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z.B auf den Seiten 103 bis 113 beschrieben.

Die reaktionskomponenten werden erfindungsgemäß nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der amerikanischen Patentschrift 2 764 565 beschrieben werden. Einzelheiten über Verarbeitungseinrichtungen, die auch erfindungsgemäß infrage kommen, werden im Kunststoff-Handbuch, Band VII, herausgegeben von

# 0 002 473

Vieweg und Höchtlen, Carl-Hanser-Verlag, München 1966, z.B. auf den Seiten 121 bis 205 beschrieben.

Bei der Schaumstoffherstellung wird erfindungsgemäß die Verschäumung oft in Formen durchgeführt. Dabei wird das Reaktiongemisch in eine Form eingetragen. Als Form-material kommt Metall, z.B. Aluminium, oder Kunststoff, z.B. Epoxidharz, in Frage. In der Form schäumt das schäumfähige Reaktionsgemisch auf und bildet den Formkörper. Die Formverschäumung kann dabei so durchgeführt werden, daß das Formteil an seiner Oberfläche Zellstruktur aufweist, es kann aber auch so durchgeführt werden, daß das Formteil eine kompakte Haut und einen zelligen Kern aufweist. Erfindungsgemäß kann man in diesem Zusammenhang so vorgehen, daß man in die Form so viel Schäumfähiges Reaktionsgemisch einträgt, daß der gebildete Schaumstoff die Form gerade ausfüllt. Man kann aber auch so arbeiten, daß man mehr schäumfähiges Reaktionsgemisch in die Form einträgt, als zur Ausfüllung des Forminneren mit Schaumstoff notwendig ist. Im letztgenannten Fall wird somit unter "overcharging" gearbeitet; eine derartige Verfahrensweise ist z.B. aus den amerikanischen Patentschriften 3 178 490 und 3 182 104 bekannt.

Bei der Formverschäumung werden vielfach an sich bekannte "äußere Trennmittel", wie Siliconöle, mitverwendet. Man kann aber auch sogenannte "innere Trennmittel", gegebenenfalls im Gemisch mit äußeren Trennmitteln, verwenden, wie sie z.B. aus den deutschen Offenlegungsschriften 2 121 670 und 2 307 589 bekanntgeworden sind.

ErfindungsgemäB lassen sich auch kalthärtende Schaumstoffe herstellen (vgl. britische Patentschrift 1 162 517, deutsche Offenlegungsschrift 2 153 086).

Selbstverständlich können aber auch Schaumstoffe durch Blockverschäumung oder nach dem an sich bekannten Doppeltransportbandverfahren hergestellt werden.

Dich ausschließliche Umsetzung der erfindungsgemäß zugänglichen Polyhydroxylverbindungen (ohne Mitverwendung anderer gegenüber Isocyanaten reaktiver Komponenten) mit stark elastifizierenden Polyisocyanaten, wie z.B. Polyisocyanaten mit Biuretstuktur (DE—A 1 543 178), führt zu harten, lichtechten, kratz- und lösungsmittelfesten Beschichtungen und Lacken.

Durch basisch oder sauer katalysierte Propoxylierung oder/und Oxyäthylierung der Polyole lassen sich ferner Polyätheralkohole hoher Funktionalität gewinnen, die in hohen OH-Zahl-Bereichen für die Herstellung von harten bzw. halbharten zellförmigen Polyurethankunststoffen und bei neidrigen OH-Zahlen als Ausgangsmaterialien für hochelastische Polyurethanschaumstoffe Verwendung finden. Bezüglich näherer Einzelheiten der Polyätherherstellung sei auf DE—A 2 639 083 verwiesen.

Die folgenden Beispiele erläutern das erfindungsgemäße Verfahren. Wenn nicht anders vermerkt, sind Mengenangaben als Gewichtsteile bzw. Gewichtsprozente zu verstehen.

Die Beispiele 3 und 4 zeigen, daß die notwendige Katalysatormenge sehr gering ist.

**Beispel 1 (Vergleichsbeispiel)**

Das Beispiel zeigt, daß bei Verfahren des Standes der Technik bei wesentlich erhöhtem Zeit- und Katalysatoraufwand Produkte mit höherem Anteil an $C_6$—$C_8$-Komponenten erhalten werden als beim erfindungsgemäßen Verfahren.

250 ml einer Formoselösung gemäß Beispiel 1 von DT—OS 2 721 186 werden zusammen mit 80 g Raney-Nickel in einem 0,7 1-Autokiaven bei 150 bar Wasserstoffdruck 4 Stunden bei 30°C, dann 1 Stunde bei 60°C und schließlich 1 Stunde bei 100°C hydriert.

Man erhält eine leicht gelbliche Lösung von Polyhydroxylverbindungen mit einem Gehalt an reduzierenden Gruppen von 0,018% und der folgenden molekularen Verteilung:

| | |
|---|---|
| Verbindungen mit 2 C-Atomen | 0,8 |
| Verbindungen mit 3 C-Atomen | 2,2 |
| Verbindungen mit 4 C-Atomen | 5,6 |
| Verbindungen mit 5 C-Atomen | 30,4 |
| Verbindungen mit 6 C-Atomen | 40,0 |
| Verbindungen 7 u. mehr | 21,0 |

**Beispiel 2 (Vergleichsbeispiel)**

Das Beispiel zeigt, daß weder die Einstellung des pH-Wertes auf einen alkalischen Wert, noch eine Temperaturerhöhung allein die niedermolekulare Komponentenverteilung der folgenden Beispiele bewirken kann.

250 ml der Formoselösung aus Beispiel 1 werden auf pH=10 eingestellt und zusammen mit 80 g Raney-Nickel in einem 0,7 1-Autoklaven bei 150 bar Wasserstoffdruck 30 min. hydriert. Während dieser Zeit wird die Temperatur von 30 auf 100°C erhöht. Man hydriert 1 Stunde bei 100°C und eine weitere Stunde bei 140°C nach. Man erhält eine farblose Lösung, in der keine reduzierenden Anteile mehr nachgewiesen werden können, mit folgender molekularer Verteilung::

| | |
|---|---|
| Verbindungen mit 2 C-Atomen | 3,2 |
| Verbindungen mit 3 C-Atomen | 8,0 |
| Verbindungen mit 4 C-Atomen | 12,6 |
| Verbindungen mit 5 C-Atomen | 33,6 |
| Verbindungen mit 6 C-Atomen | 27,0 |
| Verbindungen mit 7 u. mehr | 16,6 |

10

Allgemeine Versuchsbeschreibung der Hydrierungen
(Beispiel 3 und 4)

In einem 3 1-Edelstahlautoklaven werden 100 Teile Katalysator (K) in 300 Teilen Wasser vorgelegt und auf Hydriertemperatur (T) aufgeheizt. Dann wird das überstehende Volumen mit Wasserstoffgas bei Betriebsdruck (P) gefüllt. 500 Teile einer gemäß Beispiel 1 von DE—A 2 721 186 hergestellten 50 %igen Formoselösung mit einem Gehalt an reduzierbaren Gruppen (bestimmt als Carbonylgruppen) von 11,1% werden auf den vorgesehenen pH-Wert eingestellt und anschließend innerhalb einer festgesetzten Pumpzeit (PZ) in den Autoklaven eingepumpt. Danach wird eine festgesetzte Zeit (HZ) nachhydriert. Man läßt dann über ein Steigrohr mit Fritte, welche den Katalysator zurückhält, 500 Teile Lösung ab. Anschließend verfährt man in gleicher Weise mit der nächsten 500 Teile enthaltenden Charge Formoselösung. Es wird bis zum Nachlassen der Wirksamkeit des Katalysators hydriert, bzw. bis zur gewünschten Produktmenge oder Zyklenzahl. Ein Katalysatorverlust wurde auch nach vielen Hydrierzyklen nicht festgestellt. Die hydrierten Lösungen werden gesammelt und im Vakuum von der Hauptmenge an Wasser befreit. Man erhält in allen Fällen farblose bis leicht gelblich gefärbte Formite, die ohne weitere Reinigung weiterverarbeitet werden können. Gegebenenfalls können die Lösungen jedoch auch über Ionenaustauscher vollentsalzt werden. Die angegebenen Komponentenverteilungen wurden aus gaschromatographischen Analysen errechnet.

Beispiel 3

Das Beispiel zeigt, daß nach dem erfindungsgemäßen Verfahren mit sehr geringem Katalysatoraufwand hydriert werden kann.

pH=10,0      P=150 bar      T=100°C
PZ=25 min    HZ=25 min      K=Raney-Nickel/Eisen (85%/15%)

| Chargen-Nr | Gew.-%>C=O im Hydrierprodukt |
|---|---|
| 1 | 0,016 |
| 2 | 0,016 |
| 3 | 0,016 |
| 4 | 0,016 |
| 5 | 0,016 |
| 6 | 0,016 |
| 7 | 0,016 |
| 8 | 0,016 |
| 9 | 0,016 |
| 10 | 0,016 |
| 20 | 0,031 |
| 30 | 0,031 |
| 40 | 0,031 |
| 50 | 0,031 |
| 100 | 0,040 |
| 150 | 0,023 |
| 200 | 0,029 |
| 250 | 0,020 |
| 300 | 0,028 |
| 350 | 0,014 |
| 400 | 0,032 |

Man erhält eine farblose Lösung mit folgender molekularer Verteilung

| Verbindungen mit | |
|---|---|
| 2 C-Atomen | 3,9 Gew.-% |
| 3 C-Atomen | 20,7 Gew.-% |
| 4 C-Atomen | 24,1 Gew.-% |
| 5 C-Atomen | 22,4 Gew.-% |
| 6 C-Atomen | 22,0 Gew.-% |
| 7 u. mehr | 6,8 Gew.-% |

Beispiel 4

Das Beispiel zeigt, daß nach dem erfindungsgemäßen Verfahren mit geringem Katalysatoraufwand sehr schnell hydriert werden kann.

pH=10,5      P=150 bar      T=140°C
PZ=6 min     HZ=6 min       K=Raney-Nickel/Eisen (85%/15%)

## 0 002 473

| Chargen-Nr. | Gew.-%>C=O im Hydrierprodukt |
|---|---|
| 1 | 0,005 |
| 2 | 0,005 |
| 3 | 0,006 |
| 4 | 0,008 |
| 5 | 0,006 |
| 6 | 0,005 |
| 7 | 0,006 |
| 8 | 0,006 |
| 9 | 0,012 |
| 10 | 0,012 |
| 20 | 0,014 |
| 30 | 0,014 |
| 40 | 0,025 |
| 50 | 0,031 |
| 100 | 0,034 |
| 150 | 0,042 |
| 200 | 0,027 |
| 260 | 0,023 |

Man erhält eine farblose Lösung mit folgender molekularer Verteilung:

| | |
|---|---|
| Verbindungen mit 2 C-Atomen | 6,8 Gew.-% |
| 3 C-Atomen | 19,3 Gew.-% |
| 4 C-Atomen | 22,3 Gew.-% |
| 5 C-Atomen | 19,0 Gew.-% |
| 6 C-Atomen | 20,0 Gew.-% |
| 7 und mehr C-Atomen | 12,6 Gew.-% |

Beispiel 5

Herstellung eines Polyurethanschaumstoffs
25 Teile eines auf Äthylendiamin gestarteten Polypropylenoxids (OH-Zahl: 74),
22 Teile des Formits aus Beispiel 3,
10 Teile Trichloräthylphosphat,
15 Teile Monofluortrichlormethan,
0,5 Teile Dimethylbenzylamin,
0,5 Teile eines handelsüblichen Siliconstabilisators (L-5420 der UCC), und
75 Teile eines technischen Phosgenierungsproduktes von Anilin/Formaldehyd-Kondensaten (NCO-Gehalt: 29%)
werden intensiv vermischt und das Gemisch in einer offenen Form aufschäumen gelassen.
Man erhält einem harten, feinzelligen Schaumstoff mit großer Reißfestigkeit und Dimensionsstabilität.

### Patentansprüche

1. Verfahren zur Herstellung von niedermolekularen, mehrwertigen Alkoholen durch Reduktion von Formose bei einer Temperatur von 80 bis 220°C und unter einem Wasserstoffdruck von 50 bis 300 bar in Gegenwart eines Metallkatalysators bei einem pH-Wert von 7,5 bis 12,5, dadurch gekennzeichnet, daß man

(1) eine mindestens 20 %ige Lösung des zu reduzierenden Produkts chargenweise in einen Reaktor einbringt, so daß der Gehalt an reduzierbaren Gruppen (bestimmt als Carbonylgruppen) im Produktgemisch innerhalb des Reaktors einen Wert von 2 Gew.-% nicht überschreitet und daß jede Charge die 3-bis 30-fache Menge des im Reaktor vorhandenen Katalysators beträgt,

(2) der Katalysator stationär im Reaktor verbleibt und seine Menge $10^{-4}$ bis $5.10^{-2}$ Gewichtsteile pro 1 Gewichtsteil der Gesamtmenge sämtlicher Chargen an zu reduzierenden Ausgangsprodukten beträgt und

(3) das Reaktionsprodukt chargenweise aus dem Reaktor abzieht, nachdem der Gehalt an reduzierbaren Gruppen (bestimmt als Carbonylgruppen) unter 0,15 Gew.-% gesunken ist.

2. Verfahren nach Anspruch 1, dadurch gekennzeichnet, daß die einzelnen Chargen mit einer Geschwindigkeit zugepumpt werden, welche der Füllung von 1/6 des Reaktorvolumes in 3 bis 120 Minuten entspricht.

3. Verfahren nach Ansprüchen 1 bis 2, dadurch gekennzeichent, daß nach dem Zupumpen jeder Charge während einer Zeitdauer, die der halben bis 4-fachen Zupumpzeit entspricht, nachhydriert wird.

4. Verfahren nach Ansprüchen 1 bis 3, dadurch gekennzeichnet, daß der Formose bis zu 80 Gew.-

12

**0 002 473**

% (bezogen auf Gesamtmenge an zu hydrierenden Produkten) an weiteren natürlichen oder synthetischen Zuckern zugesetzt werden.

5. Verwendung der niedermolekularen, mehrwertigen Alkohole nach Anspruch 1 bis 4 zur Herstellung von gegebenenfalls zellförmigen Polyurethankunststoffen durch Umsetzung von

a) Polyisocyanaten mit

b) mindestens zwei aktive Wasserstoffatome aufweisenden Verbindungen mit einem Molekulargewicht zwischen 32 und 400, gegebenenfalls

c) mindestens zwei aktive Wasserstoffatome aufweisenden Verbindungen mit einem Molekulargewicht zwischen 400 und 10 000 sowie gegebenenfalls

d) Treibmitteln, Katalysatoren und weiteren an sich bekannten Zusatzstoffen,

dadurch gekennzeichnet, daß als Komponente b), gegebenenfalls nur anteilsweise, gemäß Anspruch 1 bis 4 hergestellte Gemische niedermolekularer, merhwertiger Alkohole eingesetzt werden.

## Claims

1. Process for the preparation of low molecular weight, polyhydric alcohols by the reduction of formose at a temperature of from 80 to 220°C and a hydrogen pressure of from 50 to 300 bar in the presence of a metal catalyst at a pH value of from 7.5 to 12.5, characterised in that

(1) a solution at a concentration of at least 20% of the product which is required to be reduced is introduced batch-wise into a reactor so that the proportion of reducible groups (determined as carbonyl groups) in the product mixture inside the reactor does not exceed a value of 2% by weight and each batch amounts to from 3 to 30 times the quantity of catalyst present in the reactor,

(2) the catalyst remains stationary in the reactor and the quantity of catalyst is from $10^{-4}$ to 5 times $10^{-2}$ parts by weight per 1 part by weight of the total quantity of all the batches of starting product to be reduced and

(3) the reaction product is withdrawn batch-wise from the reactor after the proportion of reducible groups (determined as carbonyl groups) has fallen below 0.15% by weight.

2. Process according to Claim 1, characterised in that the individual batches are pumped in at a rate which corresponds to filling 1/6th of the volume of the reactor in from 3 to 120 minutes.

3. Process according to Claims 1 to 2, characterised in that after each batch has been pumped in, hydrogenation is continued for a period equal to from one half to 4 times the time required for pumping in the batch.

4. Process according to Claims 1 to 3, characterised in that up to 80% by weight (based on the total quantity of products to be hydrogenated) of other natural or synthetic sugars are added to the formose.

5. Use of the low molecular weight, polyhydric alcohols according to Claims 1 to 4 for the preparation of optionally cellular polyurethane resins by the reaction of

a) polyisocyanates with

b) compounds containing at least 2 active hydrogen atoms and having a molecular weight of between 32 and 400, optionally,

c) compounds containing at least 2 active hydrogen atoms and having a molecular weight of between 400 and 10,000, and optionally

d) blowing agents, catalysts and other known additives,

characterised in that the compounds used as component b) consist, optionally only partly, of mixtures of low molecular weight, polyhydric alcohols prepared according to Claims 1 to 4.

## Revendications

1. Procédé de production d'alcools polyvalents de bas poids moléculaire par réduction de formose à une température de 80 à 220°C et sour pression d'hydrogène de 50 à 300 bars en présence d'un catalyeur métallique à un pH de 7,5 à 12,5, caractérisé en ce que:

(1) on introduit par portions dans un réacteur une solution an moins à 20% du produit à réduire, de maniére que la teneur en groups réductibles (exprimés en groupes carbonyle) dans le mélange produit à l'intérieur du réacteur ne dépasse pas une valeur de 2% en poids et que chaque portion représente 3—30 fois la quantité de catalyseur présente dans le réacteur;

(2) le catalyseur reste stationnaire dans le réacteur et sa quantité est de $10^{-4}$ à $5.10^{-2}$ partie en poids par partie en poids de la quantité totale de toutes les portions de matières de départ à réduire; et

(3) le produit réactionnel est déchargé par portions du réacteur, après que la teneur en groupes réductibles (exprimés en groupes carbonyle) s'est abaissée audessous de 0,15% en poids.

2. Procédé suivant la revendication 1, caractérisé en ce que les portions individuelles sont introduites par pompage à une vitesse qui correspond au remplissage du sixième du volume du réacteur en 3 à 120 minutes.

3. Procédé suivant les revendications 1 et 2, caractérisé en ce qu'on effectue une hydrogénation

# 0 002 473

subséquente après l'introduction par pompage de chaque portion pendant une période qui correspond à une durée allant de la moitié au quadruple de la période d'introduction par pompage.

4. Procédé suivant les revendications 1 à 3, caractérisé en ce qu'une quantité allant jusqu'à 80% en poids (par rapport à la quantité totale de produits à hydrogéner) d'autres sucres naturels ou synthétiques est ajoutée au formose.

5. Utilisation des alcools polyvalents de bas poids moléculaire suivant les revendications 1 à 4 pour la production de matières plastiques du type polyuréthanne, éventuellement cellulaires, par réaction:

    a) de polyisocyanates avec

    b) des composés portant au moins 2 atomes actifs d'hydrogène, de poids moléculaire compris entre 32 et 400, le cas échéant

    c) des composés portant au moins 2 atomes actifs d'hydrogène, de poids moléculaire compris entre 400 et 10 000 ainsi que, le cas échéant

    d) des agents porogènes, des catalyseurs et d'autres additifs connus,

caractérisée en ce qu'on utilise comme composant b), le cas échéant par portions seulement, des mélanges d'alcools polyvalents de bas poids moléculaire obtenus conformément aux revendications 1 à 4.